# EUROPÄISCHE PATENTSCHRIFT

(11) **EP 1 338 261 B1**
(45) Veröffentlichungstag und Bekanntmachung des Hinweises auf die Patenterteilung: **19.04.2006**
(21) Anmeldenummer: 03001577.0
(22) Anmeldetag: 24.01.2003
(51) Int. Cl.: A61F 13/02

(54) **Verfahren zur Herstellung eines partiell beschichteten Pflasters**
Method for producing a partially coated plaster
Procédé de production des pansements pourvus d'un revêtement partiel

(30) Priorität: 23.02.2002 DE 10207776
(43) Veröffentlichungstag der Anmeldung: 27.08.2003
(73) Patentinhaber: tesa AG, 20253 Hamburg (DE)
(72) Erfinder: Schliephacke, Ralf, 25524 Itzehoe (DE); Zimmermann, Dieter, 21635 Jork (DE)
(74) Vertreter: Stubbe, Andreas

(56) Entgegenhaltungen:
- EP-A- 0 747 027
- DE-A- 3 202 775
- DE-A- 3 423 293
- DE-A- 4 314 834
- PATENT ABSTRACTS OF JAPAN vol. 005, no. 188 (C-081), 27. November 1981 (1981-11-27) & JP 56 110613 A (LION CORP;OTHERS: 01), 1. September 1981 (1981-09-01)

## Beschreibung

Die Erfindung betrifft ein Verfahren zur Herstellung eines partiell mit einem Kontaktstoff beschichteten, zum Aufkleben auf die Haut vorgesehenen Pflasters, das mit einer durch Ziehen parallel zur Verklebungsfläche rückstandsfrei von der Haut entfembaren Haftklebemasse aufgebaut ist.

Es ist bekannt, Pflaster mit Stoffen zu versehen, die zumindest einen Teil der vom Pflaster abgedeckten Haut pflegen, in die Haut eindringen oder durch die Haut als pharmazeutischer Wirkstoff in den Körper eindringen sollen. Da diese Stoffe in einen direkten Kontakt mit der Haut gelangen, werden sie im Folgenden als Kontaktstoffe bezeichnet.

Kosmetische Pflaster dieser Art können als Reinigungspflaster dienen, indem durch die Einwirkung des Pflasters Teile des Sebums oder der in den Talgdrüsenausgängen angesammelten Verschmutzungen an dem Pflaster haften und mit diesem abgezogen und von der Haut entfernt werden. Bekannt ist ferner, in der Klebmatrix des Pflasters Wirkstoffe zu lösen oder zu dispergieren, sodass diese an die Haut abgegeben werden. Die Freisetzung der Wirkstoffe erfolgt jedoch regelmäßig nicht in dem gewünschten Maße und ist schlecht kontrollierbar.

Aus dem medizinischen Bereich sind Wirkstoffpflaster bekannt, mit deren Hilfe sich Wirkstoffe der Haut zuführen lassen.

Die genannten, vorbekannten Pflaster weisen den gemeinsamen Nachteil auf, dass sie regelmäßig ein Anfeuchten der Haut vor dem Aufkleben des Pflasters erfordern und der Vorgang des Abziehens des Pflasters am Ende der Einwirkungszeit unangenehm bis schmerzhaft sein kann.

Es ist bereits vorgeschlagen worden, Pflaster der genannten Art mit selbstklebenden Haftklebemassen aufzubauen, die sich rückstandslos und beschädigungsfrei von der Oberfläche eines Substrats entfernen lassen, indem an ihnen in Richtung der Verklebungsebene gezogen wird. Beschrieben sind solche Produkte u.a. in DE 33 31 016, WO 92/11332, WO 92/11333, DE 196 49 728, DE 196 49 729, DE 197 08 366 und DE 198 42 864. Ein für medizinische Zwecke vorgesehenes Pflaster dieser Art ist in EP 0 747 027 beschrieben, das insbesondere für eine klassische Wundabdeckung vorgesehen ist. Das Versehen der Klebeschicht mit Wirkstoffen ist in dieser Druckschrift nicht offenbart.

Durch DE 34 23 293 C2 ist es bekannt, einen Adhäsionsklebstoff eines selbstklebenden Pflasters partiell mit Wirkstoff-Segmenten zu beschichten. Dabei ist zwischen dem Wirkstoffsegment und der Klebstoffschicht jeweils ein der Form des Wirkstoffsegments angepasstes Trennfilmsegment vorgesehen, das im Druckverfahren aufgebracht wird.

Ein Pflaster der hier angesprochenen Art soll eine hohe Flexibilität aufweisen, damit es sich dem Verlauf der Hautpartien, mit denen es in Kontakt kommen soll, vollständig anpassen kann. Dies ist insbesondere problematisch für ein Pflaster, das auf Gesichtspartien aufgelegt werden soll. Die Herstellung derartiger flexiblere Pflaster und die partielle Aufbringung des Kontaktstoffes auf die Haftklebemasse erfordert bei einer sehr flexiblen Ausbildung der Haftklebemasse besondere Maßnahmen.

Der Erfindung liegt daher die Aufgabe zugrunde, ein Verfahren der eingangs erwähnten Art anzugeben, das in industriellem Maßstab die Herstellung von Pflastern erlaubt, die eine im Wesentlichen nur durch die Haftklebemasse begrenzte Flexibilität aufweisen können.

Die Lösung dieser Aufgabe gelingt bei einem Verfahren der eingangs erwähnten Art erfindungsgemäß durch folgende Verfahrensschritte:
auf eine Seite der als Bahnmaterial ausgebildeten Haftklebemasse wird ein Lack mit Release-Eigenschaft und auf die andere Seite ein abziehbares Abdeckmaterial aufgebracht,
auf den Lack wird mittels einer Klebemasse eine Hilfsfolie auflaminiert, die auf ihrer Oberseite mit einer Trennlackschicht und auf ihrer Unterseite mit einer Klebemasse versehen worden ist, wobei die Hilfsfolie mittels der Klebemasse auf den Lack laminiert wird,
beim Auflaminieren der Hilfsfolie wird das Abdeckmaterial von der Haftklebemasse entfernt und die so gebildete Bahn aufgewickelt,
eine Bahn aus leicht abziehbarem Abdeckmaterial wird einseitig mit einer partiellen Beschichtung mit dem Kontaktstoff versehen,
das beschichtete Abdeckmaterial wird auf die Haftklebemasse auflaminiert,
die Hilfsfolie wird durch Ablösen der Klebemasse von dem Lack von der Haftklebemasse entfernt,
aus der den Haftkleber aufweisenden Bahn wird das Pflaster ausgestanzt.

Das erfindungsgemäße Verfahren sieht vor, dass auf die Haftklebemasse einseitig ein Lack, vorzugsweise ein Mattlack und vorzugsweise im Druckverfahren, insbesondere Flexodruckverfahren, aufgebracht wird, der die von der Haut wegzeigende Oberfläche des Endprodukts bildet. Insbesondere für die Verwendung als Schönheitspflaster, also eines Pflasters mit hautpflegenden Eigenschaften, ist es vorteilhaft, den Lack als Mattlack auszubilden, damit eine störende glänzende Oberfläche des Pflasters vermieden wird. Aus den gleichen Gründen ist es vorteilhaft, die Haftklebemasse transparent auszubilden, um beim Auflegen auf Gesichtspartien den Eindruck einer für Maskeraden verwendeten Maske zu vermeiden.

Die andere Seite der Haftklebemasse wird zunächst mit einem Abdeckmaterial, beispielsweise einem Abziehpapier, abgedeckt. Auf diese Weise lässt sich die Haftklebemasse nach der Herstellung der von der Haut wegzeigenden Oberfläche für die weitere Verarbeitung als Bahnmaterial aufwickeln.

Für die Durchführung der weiteren erfindungsgemäßen Verfahrensschritte wird eine Hilfsfolie gebildet, die auf ihrer Oberseite mit einer Trennlackschicht versehen wird. Diese Hilfsfolie wird mittels einer Klebemasse, insbesondere einer Acrylat-Klebemasse, auf die durch den Lack gebildete Oberfläche der Haftklebemasse auflaminiert. Das dabei entstehende Bahnmaterial wird aufgewickelt, nachdem das eine gewisse Stetigkeit aufweisende Abdeckmaterial von der Haftklebemasse entfernt worden ist, sodass die Stabilität des aufgewickelten Bahnmaterials im Wesentlichen durch die Hilfsfolie bestimmt wird.

Erfindungsgemäß wird der Kontaktstoff auf eine Bahn aus einem leicht abziehbaren Abdeckmaterial einseitig in Form einer partiellen Beschichtung, beispielsweise eines regelmäßigen oder unregelmäßigen Musters, in Form von voneinander beabstandeten runden Flächen o. dgl. aufgebracht. Für dieses Aufbringen kann vorzugsweise ein rotatives Siebdruckverfahren eingesetzt werden. Nachdem der Kontaktstoff vorzugsweise auf dem Abdeckmaterial getrocknet ist, wird die Bahn aus dem beschichteten Abdeckmaterial auf die Haftklebemasse auflaminiert und die Hilfsfolie wird durch Ablösen der Klebemasse von dem Lack von der Haftklebemasse entfernt. Das Bahnmaterial kann nunmehr wieder aufgewickelt oder unmittelbar weiterverarbeitet werden, indem aus der den Haftkleber aufweisenden Bahn das Pflaster ausgestanzt wird.

Durch die erfindungsgemäß eingesetzte Hilfsfolie, die auf ihrer der Haftklebemasse wegzeigenden Oberfläche mit einem Trennlack versehen sind, lässt sich die Haftklebemasse zusammen mit der Hilfsfolie als Bahnmaterial aufwickeln, wobei der Trennlack ein Verkleben der Windungen der Wicklung durch die Haftklebemasse verhindert. Das die Haftklebemasse aufweisende Bahnmaterial kann anschließend mit dem mit dem Kontaktstoff beschichteten Abdeckmaterial laminiert werden, da die Hilfsfolie für eine ausreichende Stabilität zur Erzeugung des für das Laminieren benötigten Gegendrucks sorgt. Durch das Aufbringen des Abdeckmaterials wird nunmehr auf der Wirkseite der Haftklebemasse die erforderliche Stabilität erzeugt, sodass gleichzeitig oder später die Hilfsfolie entfernt werden kann.
Bei dem erfindungsgemäßen Verfahren wird der Kontaktstoff somit im Transfer-Verfahren auf die Haftklebemasse aufgebracht, wobei der Transferträger als "verlorener Träger" zugleich das Abdeckmaterial für die Haftklebemasse bildet, sodass in sehr rationeller und einfacher Weise das gewünschte Produkt mit einer dekorativen, von der Haut wegzeigenden Oberfläche und einem leicht abziehbaren Abdeckmaterial auf der zur Haut zeigenden Seite der Haftklebemasse versehen ist.

Die Erfindung soll im Folgenden anhand der beigefügten Zeichnung näher erläutert werden. Es zeigen:
- Figur 1:: einen Produktaufbau nach einem ersten Arbeitsgang
- Figur 2:: einen Aufbau einer Hilfsfolie
- Figur 3:: einen Produktaufbau nach dem Auflaminieren der Hilfsfolie
- Figur 4:: einen Produktaufbau nach dem Auflaminieren eines mit dem Kontaktstoff beschichteten Abdeckmaterials
- Figur 5:: den Aufbau des Endprodukts nach dem Entfernen der Hilfsfolie
- Figur 6:: eine schematische Darstellung eines Antifaltenpflasters
- Figur 7:: eine schematische Darstellung eines weiteren Antifaltenpflasters
- Figur 8:: eine schematische Darstellung eines Tränensackpflasters und
- Figur 9:: eine schematische Darstellung eines weiteren Tränensackpflasters.

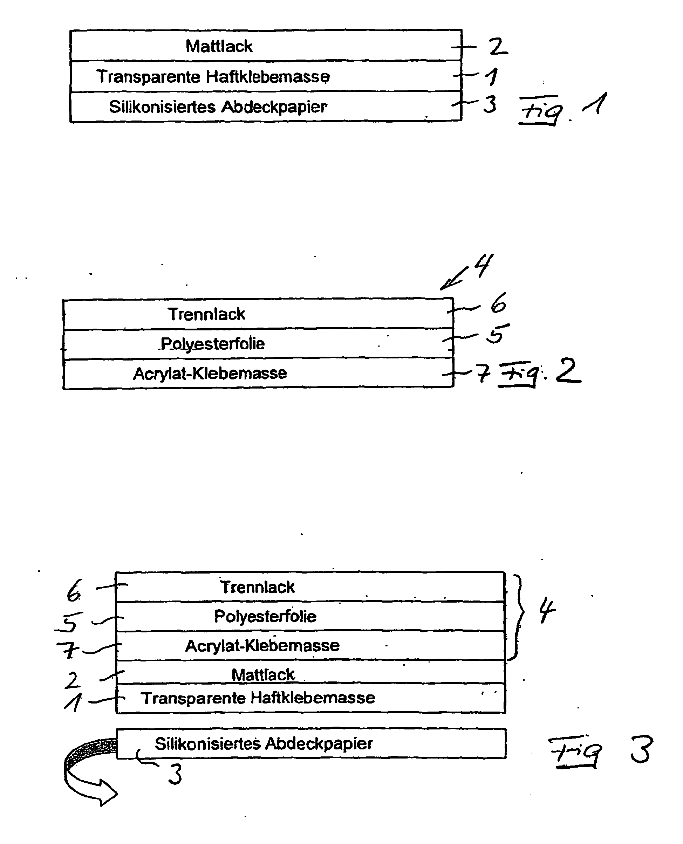

Gemäß Figur 1 wird zunächst ein Streifen einer transparenten Haftklebemasse 1 hergestellt. Die Herstellung kann vorzugsweise gemäß der DE 100 03 318 A1, dort vorzugsweise gemäß Beispiel I-8 erfolgen, und zwar in einer Dicke von 0,3 mm und mit einem Flächengewicht von ca. 300 g/m².

Die hergestellte transparente Haftklebemasse 1 wird auf der einen Seite mit einem Lack 2 in Form eines Mattlacks beschichtet Zum Einsatz kann hierbei ein kationisches aushärtender UV-Mattlack der Firma SICPA/Aarberg, kommen. Durch Beimischung von 5 Gew. % Zylinderabstoßmittel wird die Druckfarbe für die Verarbeitung optimiert. Mittels einer UV-Flexdruckmaschine ARSOMAem 410 wird die Haftklebemasse 1 bei einer Maschinengeschwindigkeit von 30 m/min über eine Flexodruckstation bedruckt. Die genau definierte Farbübertragung auf das Flexodruckklischee erfolgt mittels einer entsprechenden Rasterwalze im Negativ-Rakelverfahren. Es erfolgt danach die Farbübertragung vom Klischee auf die Haftklebemasse 1 in einer Farbmenge von 10 g/m². Der Einsatz des Flexodruck-Verfahrens erlaubt eine definierte Farbübertragung mit nur einem kurzzeitigen Kontakt, der ein Ankleben des Druckklischees an der Haftklebemasse 1 vermeidet.

Der Farbauftrag auf der Haftklebemasse 1 wird durch leistungsstarke UV-Strahlerröhren ausgehärtet. Hierfür wurde eine UV-Station Micro EV-Station GEW mit einer Strahlerleistung von 110 W/cm und einer Wellenlänge von 365 nm eingesetzt.

Auf die selbstklebende andere Seite der Haftklebemasse 1 wird als Abdeckmaterial 3 ein silikonisiertes Abdeckpapier mit einem Flächengewicht von ca. 70 g/m² aufgebracht.

Im zweiten Arbeitsgang wird eine Hilfsfolie 4 (Figur 2) hergestellt, indem eine Polyesterfolie 5 mit einer Stärke von ca. 25 µm auf einer Seite mit einem Trennlack 6 mit einem Flächengewicht von ca. 0,5 g/m² auf der Basis von Polydimethylsiloxan aufgebracht. Dieser Trennlack hat sog. Release-Eigenschaft gegenüber der Haftklebemasse 1.

Die Polyesterfolie 5 wird auf der anderen Seite mit einer Klebemasse 7, vorzugsweise einer Acrylat-Klebemasse mit ca. 10 g/m² beschichtet Diese Klebemasse 7 wird so eingestellt, dass sie nur eine leichte Klebwirkung hat.

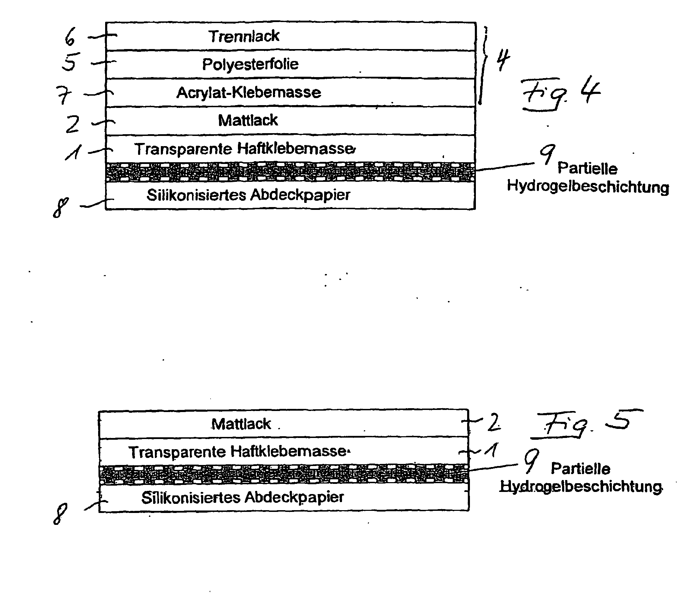

Bei dem in Figur 3 dargestellten Arbeitsgang wird die gemäß Figur 2 hergestellte Hilfsfolie 4 auf den Produktaufbau gemäß Figur 1 auflaminiert, indem die Klebemasse 7 auf den Lack. 2 der Haftklebemasse 1 laminiert wird. Im gleichen Arbeitsgang wird das Abdeckmaterial 3 von der Haftklebemasse abgezogen. Das so gebildete Bahnmaterial wird aufgewickelt, wobei der Trennlack 6 an der freien Seite der Haftklebemasse 1 anliegt und eine Klebwirkung der Haftklebemasse auf dieser Seite verhindert.

Figur 4 verdeutlicht, dass ein Abdeckmaterial 8, das wieder in Form eines silikonisierten Abdeckpapiers mit einem Flächengewicht von ca. 70 g/m² ausgebildet sein kann, mit einem Kontaktstoff 9, der vorzugsweise ein Hydrogel mit einem Flächengewicht von ca. 10 bis 50 g/m² ist, beschichtet wird. Die schematische Darstellung in Figur 4 soll symbolisieren, dass nur ein Teil der Oberfläche des Abdeckmaterials 8 mit dem Kontaktstoff 9 beschichtet ist. Der als wasserbasierendes Hydrogel ausgebildete Kontaktstoff 9 wird über ein rotatives Siebdruckverfahren auf das Abdeckmaterial 8 aufgebracht und einer anschließenden Trocknung in einem Trockenkanal unterzogen. Die Trocknung kann dabei mit konventionellen Konvektions- und/oder Infrarot-Trocknungssystemen vorgenommen werden. Nach der Trocknung wird das beschichtete Abdeckmaterial 8 auf das gemäß Figur 3 hergestellte Bahnmaterial so laminiert, dass die partielle Beschichtung mit dem Kontaktstoff 9 auf die transparente Haftklebemasse 1 aufgebracht wird.

Anschließend wird die Hilfsfolie 4 entfernt, indem die Klebemasse von dem Mattlack 2 abgelöst wird. Da die Klebemasse 7 auf dem Lack 2 nur schwach klebend ausgebildet ist, macht diese Delaminierung keine Schwierigkeiten. Es entsteht somit das in Figur 5 dargestellte Endprodukt mit der Haftklebemasse 1, die auf der von der Haut abgewandten Seite mit dem Lack 2 abgedeckt ist und auf deren andere Seite der Kontaktstoff 9 im Transfer-Verfahren aufgebracht ist, wobei das Abdeckmaterial 8 als verlorener Träger für den Transfervorgang verwendet worden ist und an dem Produkt verbleibt, bis es beim Endkunden durch Abziehen des Abdeckmaterials 8 zur Anwendung gelangt. Nach dem Abziehen des Abdeckmaterials 8 liegt die zum Kontakt mit der Haut vorgesehene Seite der Haftklebemasse 1, partiell beschichtet mit dem Kontaktstoff 9, zur Auflage auf der Haut frei.

Figur 5 zeigt den Aufbau des Endprodukts als Bahnmaterial. Das eigentliche Endprodukt in Form eines Pflasters wird aus dem Bahnmaterial der Figur 5 ausgestanzt und vereinzelt, wobei die mit dem Lack 2 versehene Haftklebemasse 1 mit der partiellen Beschichtung mit dem Kontaktstoff 9 in der Außenkontur kleiner ist als das Abdeckmaterial 8.

Als Kontaktstoff 9 wird vorzugsweise ein Hydrogel mit folgender Zusammensetzung verwendet:

| | Gew. % |
|---|---|
| Wasser VES | ad 100 |
| Glycerin 85%ig | 4,00 |
| Phenoxyethanol | 0,40 |
| Phenoxyethanol + Methyldibromo Glutaronitril (Euxyl K 400) | 0,05 |
| Natronlauge 10%ig | 1,65 |
| Carbomer (Carbopol 2984) | 4,50 |
| Carbomer (Carbopol 5984) | 2,50 |

Das Auftragsgewicht des Hydrogels im fertigen Produkt beträgt ca. 10 bis 50 g/m².

Das erfindungsgemäß erhaltene Pflaster ist transparent und auf die Haut geklebt nahe unsichtbar.

Das erfindungsgemäße Verfahren lässt sich - wie beschrieben - unproblematisch mit üblichen Laminier- und Delaminiervorrichtungen und mit üblichen Druckeinrichtungen ausführen. Die erfindungsgemäße Kombination der Verfahrensschritte erlaubt eine sichere und störungsunanfällige Herstellung von hochflexiblen Pflastern.

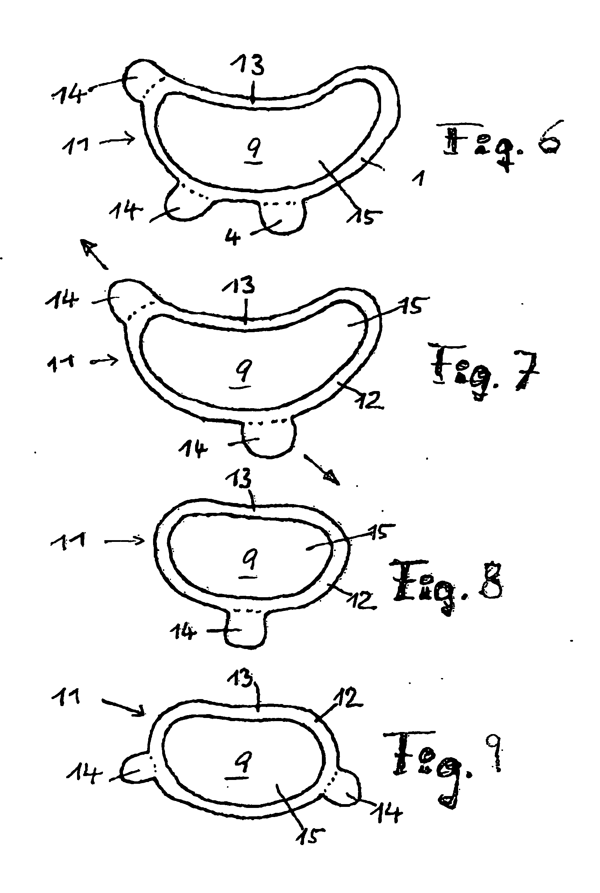

Beispiele von Ausführungsformen erfindungsgemäß hergestellter Pflaster 11 sind in den Figuren 6 bis 9 dargestellt. Sie zeigen jeweils ein Pflaster 11 mit der Haftklebemasse 1, die im Randbereich 13 für eine Verklebung auf der Haut offenliegt, nachdem das Abdeckmaterial 8 (Figur 5) abgezogen worden ist Nicht klebend ausgerüstete Anfasser 14 sind vorgesehen, um an diesen mittels vertreckendem Ziehen in Richtung der Verklebungsebene das Pflaster 11 nach Gebrauch wieder zu lösen, etwa so, wie dies durch die Pfeile A in Figur 7 angedeutet ist In seinem mittleren Bereich 15 ist die Haftklebemasse 1 mit dem Kontaktstoff 9, vorzugsweise in Form eines Hydrogels, dem kosmetische und/oder dermatologische Wirk- und/oder Hilfsstoffe beigefügt sein können, beschichtet.

Auf der in den Figuren 6 bis 9 nicht dargestellten Rückseite des Pflasters befindet sich der Lack 2 in Form eines Mattlacks.

Die in den Figuren 6 bis 9 dargestellten verschiedenen Formgebungen dienen der Anpassung an den Hautbereich, auf den das jeweilige Pflaster 11 aufgebracht werden soll.

## Patentansprüche

1. Verfahren zur Herstellung eines partiell mit einem Kontaktstoff (9) beschichteten, zum Aufkleben auf Haut vorgesehenen Pflasters, das mit einer durch Ziehen parallel zur Verklebungsfläche rückstandsfrei von der Haut entfembaren Haftklebemasse (1) aufgebaut ist,
wobei der Kontaktstoff (9) zumindest einen Teil der vom Pflaster abgedeckten Haut pflegt, in die Haut eindringt oder durch die Haut als pharmazeutischer Wirkstoff in den Körper eindringt,
**gekennzeichnet durch** folgende Verfahrensschritte:
- auf eine Seite der als Bahnmaterial ausgebildeten Haftklebemasse (1) wird ein Lack (2) mit Release-Eigenschaft und auf die andere Seite ein abziehbares Abdeckmaterial (3) aufgebracht,
- auf den Lack (2) wird eine Hilfsfolie (4) auflaminiert, die auf ihrer Oberseite mit einer Trennlackschicht (6) und auf ihrer Unterseite mit einer Klebemasse (7) versehen worden ist, wobei die Hilfsfolie mittels der Klebemasse (7) auf den Lack laminiert wird,
- beim Auflaminieren der Hilfsfolie (4) wird das Abdeckmaterial (3) von der Haftklebemasse (1) entfernt und die so gebildete Bahn aufgewickelt,
- eine Bahn aus leicht abziehbarem Abdeckmaterial (8) wird einseitig mit einer partiellen Beschichtung mit dem Kontaktstoff (9) versehen,
- das beschichtete Abdeckmaterial (8) wird auf die Haftklebemasse (1) auflaminiert,
- die Hilfsfolie (4) wird **durch** Ablösen der Klebemasse (7) von dem Lack (2) von der Haftklebemasse (1) entfernt,
- aus der die Haftklebemasse (1) aufweisenden Bahn wird das Pflaster ausgestanzt.

2. Verfahren nach Anspruch 1, **dadurch gekennzeichnet, dass** als auf die Haftklebemasse (1) aufzubringender Lack (2) ein Mattlack verwendet wird.

3. Verfahren nach Anspruch 1 oder 2, **dadurch gekennzeichnet, dass** der Lack (2) in einem Druckverfahren aufgebracht wird.

4. Verfahren nach Anspruch 3, **dadurch gekennzeichnet, dass** der Lack (2) im Flexodruckverfahren aufgebracht wird.

5. Verfahren nach einem der Ansprüche 1 bis 4, **dadurch gekennzeichnet, dass** die beiden Beschichtungen der Hilfsfolie (4) in einem Arbeitsgang vorgenommen werden.

6. Verfahren nach einem der Ansprüche 1 bis 5, **dadurch gekennzeichnet, dass** als Kontaktstoff (9) ein Hydrogel verwendet wird.

7. Verfahren nach Anspruch 6, **dadurch gekennzeichnet, dass** das Hydrogel auf dem Abdeckmaterial (8) getrocknet wird, bevor das Abdeckmaterial (8) auf die Haftklebemasse (1) auflaminiert wird.

8. Verfahren nach einem der Ansprüche 1 bis 7, **gekennzeichnet durch** die Verwendung einer transparenten Haftklebemasse.

## Claims

1. Process for producing a plaster which is partially coated with a contact substance (9), is intended for adhesion to skin and is constructed with a pressure-sensitive adhesive (1) removable from the skin without residue by pulling parallel to the bonding area, the contact substance (9) providing care treatment to at least part of the skin covered by the plaster, penetrating into the skin or penetrating through the skin, as an active pharmaceutical substance, into the body,
**characterized by** the following process steps:
- on one side of the pressure-sensitive adhesive (1) in web material form a varnish (2) having release characteristics and on the other side a peelable liner material (3) are applied,
- laminated onto the varnish (2) is an auxiliary sheet (4) provided on its top face with a release varnish layer (6) and on its bottom face with an adhesive (7), the auxiliary sheet being laminated onto the varnish by means of the adhesive (7),
- when the auxiliary sheet (4) is laminated on the liner material (3) is removed from the pressure-sensitive adhesive (1) and the web thus formed is wound up,
- a web of easily peelable liner material (8) is provided on one side with a partial coating with the contact substance (9),
- the coated liner material (8) is laminated onto the pressure-sensitive adhesive (1),
- the auxiliary sheet (4) is removed from the pressure-sensitive adhesive (1) by detaching the adhesive (7) from the varnish (2),
- the plaster is die-cut from the web bearing the pressure-sensitive adhesive (1).

2. Process according to Claim 1, **characterized in that** a matt varnish is used as varnish (2) for application to the pressure-sensitive adhesive (1).

3. Process according to Claim 1 or 2, **characterized in that** the varnish (2) is applied in a printing process.

4. Process according to Claim 3, **characterized in that** the varnish (2) is applied in the flexographic printing process.

5. Process according to one of Claims 1 to 4,
**characterized in that** the two coatings of the auxiliary sheet (4) are performed in one operation.

6. Process according to one of Claims 1 to 5,
**characterized in that** a hydrogel is used as contact substance (9).

7. Process according to Claim 6, **characterized in that** the hydrogel is dried on the liner material (8) before the liner material (8) is laminated onto the pressure-sensitive adhesive (1).

8. Process according to one of Claims 1 to 7,
**characterized by** the use of a transparent pressure-sensitive adhesive.

## Revendications

1. Procédé de fabrication d'un pansement partiellement revêtu d'une substance de contact (9), prévu pour être appliqué sur la peau et doté d'une substance adhésive (1) apte à être retirée sans résidu de la peau par traction parallèle à la surface d'application,
dans lequel la substance de contact (9) soigne au moins une partie de la peau recouverte par le pansement, pénètre dans la peau ou pénètre dans le corps à travers la peau en tant que produit actif pharmaceutique,
**caractérisé par** les étapes qui consistent à :
- apporter un vernis antiadhésif (2) sur l'un des côtés de la substance adhésive (1) réalisée comme matériau en bande et un matériau de recouvrement (3) apte à être retiré sur l'autre côté,
- recouvrir le vernis (2) d'un film auxiliaire (4) dont le côté supérieur présente une couche de vernis de séparation (6) et le côté inférieur une substance adhésive (7), le film auxiliaire étant appliqué sur le vernis au moyen de la substance adhésive (7),
- retirer le matériau de recouvrement (3) de la substance adhésive (1) lors de l'application du film auxiliaire (4) et enrouler la bande ainsi formée,
- recouvrir partiellement avec la substance de contact (9) un côté de la bande en matériau de recouvrement (8) qui peut facilement être retiré,
- stratifier le matériau de recouvrement (8) revêtu sur la substance adhésive (1),
- retirer le film auxiliaire (4) de la substance adhésive (1) en séparant la substance adhésive (7) du vernis (2) et
- estamper le pansement dans la bande qui présente la pâte adhésive (1).

2. Procédé selon la revendication 1, **caractérisé en ce que** le vernis (2) appliqué sur la substance adhésive (1) est un vernis mat.

3. Procédé selon les revendications 1 ou 2,
**caractérisé en ce qu'**on applique le vernis (2) par impression.

4. Procédé selon la revendication 3, **caractérisé en ce qu'**on applique le vernis (2) par un procédé flexographique.

5. Procédé selon l'une des revendications 1 à 4,
**caractérisé en ce qu'**on les deux revêtements du film auxiliaire (4) sont réalisés en une étape de travail.

6. Procédé selon l'une des revendications 1 à 5,
**caractérisé en ce que** la substance de contact (9) utilisée est un hydrogel.

7. Procédé selon la revendication 6, **caractérisé en ce qu'**on sèche l'hydrogel sur le matériau de recouvrement (8) avant d'appliquer le matériau de recouvrement (8) sur la substance adhésive (1).

8. Procédé selon l'une des revendications 1 à 7,
**caractérisé par** l'utilisation d'une substance adhésive transparente.
